(19) Europäisches Patentamt
European Patent Office
Office européen des brevets

(11) Publication number: 0 552 418 A1

(12) **EUROPEAN PATENT APPLICATION**

(21) Application number: 92117193.0

(22) Date of filing: 08.10.92

(51) Int. Cl.5: C07C 233/09, A61K 31/16, A01N 37/18, C07D 295/185, A61K 31/445, A01N 43/40, C07D 333/20

(30) Priority: 13.01.92 JP 4147/92
19.06.92 JP 161044/92

(43) Date of publication of application:
28.07.93 Bulletin 93/30

(84) Designated Contracting States:
ES

(71) Applicant: TAKEDA CHEMICAL INDUSTRIES, LTD.
1-1, Doshomachi 4-chome
Chuo-ku, Osaka 541(JP)

(72) Inventor: Sugihara, Yoshihiro
202, 7-9, Kasuga 1-chome, Tsukuba
Ibaraki 305(JP)
Inventor: Nakamichi, Masanari
152-20, Higashisonodacho 4-chome
Amagasaki, Hyogo 661(JP)
Inventor: Igarashi, Akira
2-315, 18 nakayamasakuradai 6-chome
Takarazuka, Hyogo 665(JP)

(74) Representative: von Kreisler, Alek et al
Patentanwälte von Kreisler Selting Werner
Deichmannhaus am Hauptbahnhof
W-5000 Köln 1 (DE)

(54) **N-substituted-3-iodopropiolamide, production and use thereof.**

(57) A novel amide compound, more particularly N-substituted-3-iodopropiolamide compound, which exhibits excellent antibacterial, antifungal, insecticidal, antialgal, miticidal and anti-termite activity; a method for producing the amide compound; and a noxious organisms controlling agent containing said amide compound.

EP 0 552 418 A1

## BACKGROUND OF THE INVENTION

The present invention relates to a novel amide compound, more particularly to a N-substituted-3-iodopropiolamide compound, a method for producing the compound and a noxious organisms controlling agent containing said compound.

Yamazoe et al. have reported that ester- or amide-derivatives of propiolic acid have fungistatic activity [Japanese J. Pharmacology, 102 278 (1982)]. For N-(substituted phenyl)propiolamide, germicidal activity, wood preserving activity or underwater organisms repellent activity is known [cf. Japanese Patent Publication Nos. 14756/1965, 31220/1969 and 58885/1985, respectively]. Further, antibacterial and antifungal activity of 3-iodopropiolic acid (I-C≡C-COOH), esters, and analogues thereof has been reported [Ann. Applied Biol., 36 250 (1949); Japanese J. Pharmacology, 90 1578 (1970)]. On the other hand, 3-iodopropiolamide (I-C≡C-$CONH_2$) is known, but application thereof has not been described [Chemical Abstract, 81 135429q (1974)].

The compounds described in the above literature and patents are insufficient because of their insufficient activity. Accordingly, it is necessary to provide a compound having an excellent antibacterial and antifungal activity.

## SUMMARY OF THE INVENTION

The present inventors have studied intensively to attain the above object. As the results, the inventors have succeeded in preparing novel N-substituted-3-iodopropiolamide compound having an excellent antibacterial and antifungal activity and have found that said compounds also exhibit antialgal, insecticidal, miticidal, anti-termite activity. According to further investigation, the inventors have attained the present invention.

## DESCRIPTION OF THE PREFERRED EMBODIEMENT

The present invention relates to a N-substituted-3-iodopropiolamide compound represented by the formula:

I-C≡C-CO-$NR_1R_2$ [I]

wherein $R_1$ is a hydrocarbon group which may be substituted or an aromatic heterocyclic group which may be substituted, $R_2$ is a hydrogen atom, a hydrocarbon group which may be substituted or an aromatic heterocyclic group which may be substituted, or $R_1$ and $R_2$ together with the adjacent nitrogen atom form a ring; a method for producing the said compound; and a noxious organisms controlling agent containing said compound.

In the compound [I] of the present invention, $R_1$ is a hydrocarbon group which may be substituted, or an aromatic heterocyclic group which may be substituted, $R_2$ is a hydrogen atom, a hydrocarbon group which may be substituted or an aromatic heterocyclic group which may be substituted, or $R_1$ and $R_2$ together with the adjacent nitrogen atom form a ring. Such hydrocarbon group includes, for example, an alkyl group, a cycloalkyl group, an alkenyl group, an alkynyl group, an aryl group and an aralkyl group.

As alkyl group, $C_{1-24}$ straight or branched chain alkyl groups are preferred. For example, methyl, ethyl, n-propyl, isopropyl, n-butyl, isobutyl, sec-butyl, tert-butyl, n-pentyl, isoamyl, tert-amyl, n-hexyl, isohexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-eicosyl, n-docosyl, n-tetracosyl and the like can be used. As alkyl groups, $C_{3-18}$ straight or branched chain alkyl groups are preferred. Those having 4 to 10 carbon atoms, such as n-butyl, isobutyl, sec-butyl, tert-butyl, n-pentyl, isoamyl, tert-amyl, n-hexyl, isohexyl, n-heptyl, n-octyl, n-nonyl, n-decyl, are most preferable. These alkyl groups may be substituted. Such substituents include, for example, cycloalkyl such as cyclopentyl, cyclohexyl; halogen such as fluorine, chlorine, bromine, iodine; cyano; hydroxy; $C_{1-4}$ alkoxy such as methoxy, ethoxy, propoxy, butoxy; carboxyl; $C_{1-4}$ alkoxycarbonyl such as methoxycarbonyl, ethoxycarbonyl, propoxycarbonyl, butoxycarbonyl; ketone; nitro; amino; carbamoyl; $C_{1-4}$ alkanoyl such as acetyl, propionyl, butyryl; aromatic heterocyclic group such as furyl (2-, 3-), thienyl (2-, 3-), pyridyl (2-, 3-, 4-), thiazolyl, imidazolyl, benzothiazolyl, benzoimidazolyl, which may be further substituted with group such as $C_{1-4}$ alkyl such as methyl, ethyl, propyl, butyl; cycloalkyl such as cyclopentyl, cyclohexyl; halogen such as fluorine, chlorine, bromine, iodine; cyano; hydroxy; $C_{1-4}$ alkoxy such as methoxy, ethoxy, propoxy, butoxy; carboxyl; $C_{1-4}$ alkoxycarbonyl such as methoxycarbonyl, ethoxycarbonyl, propoxycarbonyl, butoxycarbonyl; nitro; amino; carbamoyl; $C_{1-4}$ alkanoyl such as acetyl,

propionyl, butyryl.

As cycloalkyl group, those having 3 to 8 carbon atoms are preferred. For example, cyclopropyl, cyclobutyl, cyclopentyl, cyclohexyl, cycloheptyl, cyclooctyl. Those having 5 to 7 carbon atoms, such as cyclopentyl, cyclohexyl, cycloheptyl, are more preferred. These cycloalkyl groups may be substituted. The substituents include, for example, $C_{1-4}$ alkyl such as methyl, ethyl, propyl, butyl; halogen such as fluorine, chlorine, bromine, iodine; cyano; hydroxy; $C_{1-4}$ alkoxy such as methoxy, ethoxy, propoxy, butoxy; carboxyl; $C_{1-4}$ alkoxycarbonyl such as methoxycarbonyl, ethoxycarbonyl, propoxycarbonyl, butoxycarbonyl; ketone; nitro; amino; carbamoyl; $C_{1-4}$ alkanoyl such as acetyl, propionyl, butyryl.

As the alkenyl groups, $C_{2-24}$ straight or branched chain alkenyl groups are preferred. For example, vinyl, propenyl (1-, 2-), butenyl (1-, 2-, 3-), pentenyl, octenyl, butadienyl (1,3-) and the like. These alkenyl groups may be substituted. The substituents include, for example, cycloalkyl such as cyclopentyl, cyclohexyl; halogen such as fluorine, chlorine, bromine, iodine; cyano; hydroxy; $C_{1-4}$ alkoxy such as methoxy, ethoxy, propoxy, butoxy; carboxyl; $C_{1-4}$ alkoxycarbonyl such as methoxycarbonyl, ethoxycarbonyl, propoxycarbonyl, butoxycarbonyl; ketone; nitro; amino; carbamoyl; $C_{1-4}$ alkanoyl such as acetyl, propionyl, butyryl.

As the alkynyl groups, $C_{2-24}$ straight or branched chain alkynyl groups are preferred. For example, ethynyl, propynyl (1-, 2-), butynyl (1-, 2-, 3-), pentynyl, octynyl, decynyl and the like can be used. These alkynyl groups may be substituted. The substituents include, for example, cycloalkyl such as cyclopentyl, cyclohexyl; halogen such as fluorine, chlorine, bromine, iodine; cyano; hydroxy; $C_{1-4}$ alkoxy such as methoxy, ethoxy, propoxy, butoxy; carboxyl; $C_{1-4}$ alkoxycarbonyl such as methoxycarbonyl, ethoxycarbonyl, propoxycarbonyl, butoxycarbonyl; ketone; nitro; amino; carbamoyl; $C_{1-4}$ alkanoyl such as acetyl, propionyl, butyryl.

As the aryl groups, mono- to tricyclic aromatic hydrocarbon groups are preferred. For example, unsubstituted or substituted phenyl and naphthyls can be used. Particularly, unsubstituted or substituted phenyl is preferred. The substituents include, for example, $C_{1-4}$ alkyl such as methyl, ethyl, propyl, butyl, wherein the alkyl group may be substituted with halogen such as fluorine, chlorine, bromine, iodine; cycloalkyl such as cyclopentyl, cyclohexyl; halogen such as fluorine, chlorine, bromine, iodine; cyano; hydroxy; $C_{1-4}$ alkoxy such as methoxy, ethoxy, propoxy, butoxy; carboxyl; $C_{1-4}$ alkoxycarbonyl such as methoxycarbonyl, ethoxycarbonyl, propoxycarbonyl, butoxycarbonyl; nitro; amino; carbamoyl; $C_{1-4}$ alkanoyl such as acetyl, propionyl, butyryl. As substituents of the aryl groups, halogen is more preferred. The example of the substituted aryl groups include, for example, 2-fluorophenyl, 3-fluorophenyl, 4-fluorophenyl, 2-chlorophenyl, 3-chlorophenyl, 4-chlorophenyl, 3-bromophenyl and the like. The phenyl groups which are substituted with chlorine or fluorine, such as 3-fluorophenyl, 4-fluorophenyl, 3-chlorophenyl, 4-chlorophenyl, are more preferred.

As the aralkyl groups, $C_{1-4}$ alkyl groups substituted with mono- to tricyclic aromatic hydrocarbon groups are preferred. For example, benzyl, 2-phenylethyl, 1-phenylethyl, 3-phenylpropyl, 4-phenylbutyl, 1-naphthylmethyl, 2-naphthylmethyl and the like can be used. Particularly, benzyl, 2-phenylethyl, 1-phenylethyl, 3-phenylpropyl are preferred. These aralkyl groups may be substituted. The substituents include, for example, $C_{1-4}$ alkyl such as methyl, ethyl, propyl, butyl; cycloalkyl such as cyclopentyl, cyclohexyl; halogen such as fluorine, chlorine, bromine, iodine; cyano; hydroxy; $C_{1-4}$ alkoxy such as methoxy, ethoxy, propoxy, butoxy; carboxyl; $C_{1-4}$ alkoxycarbonyl such as methoxycarbonyl, ethoxycarbonyl, propoxycarbonyl, butoxycarbonyl; nitro; amino; carbamoyl; $C_{1-4}$ alkanoyl such as acetyl, propionyl, butyryl.

The aromatic heterocyclic group as $R_1$ or $R_2$ means a group which can be obtained by removing a hydrogen atom binding to ring-constituting carbon atoms of the aromatic heterocyclic compound. As such aromatic heterocyclic compound, 5- to 10-membered aromatic heterocyclic compounds having, in addition to carbon atoms, 1 to 4 nitrogen atoms and/or oxygen atoms and/or sulfur atoms as ring-constituting atoms are preferred. The compound may be further condensed with an aromatic hydrocarbon or an aromatic heterocyclic group. The example of the aromatic heterocyclic groups include, for example, furyl (2-, 3-), thienyl (2-, 3-), pyridyl (2-, 3-, 4-), thiazolyl, imidazolyl, benzothiazolyl, benzoimidazolyl and the like. These aromatic heterocyclic groups may be substituted. The substituents include, for example, $C_{1-4}$ alkyl such as methyl, ethyl, propyl, butyl; cycloalkyl such as cyclopentyl, cyclohexyl; halogen such as fluorine, chlorine, bromine, iodine; cyano; hydroxy; $C_{1-4}$ alkoxy such as methoxy, ethoxy, propoxy, butoxy; carboxyl; $C_{1-4}$ alkoxycarbonyl such as methoxycarbonyl, ethoxycarbonyl, propoxycarbonyl, butoxycarbonyl; nitro; amino; carbamoyl; $C_{1-4}$ alkanoyl such as acetyl, propionyl, butyryl; aryl such as phenyl, naphthyl.

When $R_1$ and $R_2$ together with the adjacent nitrogen atom form a ring, that is, a ring containing a nitrogen atom, said nitrogen-containing ring is preferably 5- to 8-membered ring. The ring-constituting atoms, other than said nitrogen atom, include carbon and/or nitrogen and/or oxygen and/or sulfur atoms.

The number of the hetero atoms constituting the ring, such as nitrogen atom, oxygen atom, sulfur atom, is preferably 1 to 4 in total. Further, said nitrogen-containing ring may be condensed with another ring. When $R_1$ and $R_2$ together with the nitrogen atom form a ring, the $-NR_1R_2$ group itself constitutes the cyclic amino group. The example of said cyclic amino group ($-NR_1R_2$) includes, for example, 1-pyrrolidyl, 1-imidazolyl, piperidino (1-piperidyl), 1-piperazinyl, 3-oxazolidinyl, hexamethylenimino, heptamethylenimino, morpholino (4-morpholinyl), 1-indolinyl. These cyclic amino groups may be substituted. The substituents include, for example, $C_{1-4}$ alkyl such as methyl, ethyl, propyl, butyl; cycloalkyl such as cyclopentyl, cyclohexyl; halogen such as fluorine, chlorine, bromine, iodine; cyano; hydroxy; $C_{1-4}$ alkoxy such as methoxy, ethoxy, propoxy, butoxy; carboxyl; $C_{1-4}$ alkoxycarbonyl such as methoxycarbonyl, ethoxycarbonyl, propoxycarbonyl, butoxycarbonyl; ketone; nitro; amino; carbamoyl; $C_{1-4}$ alkanoyl such as acetyl, propionyl, butyryl; aryl such as phenyl, naphthyl.

The compounds [I] wherein $R_1$ is an alkyl group and $R_2$ is a hydrogen atom or an alkyl group are preferred. In this case, $C_{1-18}$ alkyl groups are more preferred as such alkyl group, and $C_{4-12}$ alkyl groups, such as n-butyl, isobutyl, sec-butyl, tert-butyl, n-pentyl, isoamyl, tert-amyl, n-hexyl, isohexyl, n-heptyl, n-octyl, n-nonyl, n-decyl, n-undecyl, n-dodecyl, are especially more preferred. Among them, those having 4 to 10 carbon atoms, such as n-butyl, isobutyl, sec-butyl, tert-butyl, n-pentyl, isoamyl, tert-amyl, n-hexyl, isohexyl, n-heptyl, n-octyl, n-nonyl, n-decyl, are most preferred.

The compound [I] of the present invention can be produced by the following processes (1) to (3).

$$(1)\quad \underset{[II]}{H-C\equiv C-CO-NR_1R_2} \rightarrow \underset{[I]}{I-C\equiv C-CO-NR_1R_2}$$

This process comprises iodination of propiolamide compound [II] to produce the compound [I] of the present invention. More particularly, this process provides the compound [I] by dissolving the compound [II] in an organic solvent and reacting with an iodination agent. As such organic solvent, those inert for iodination agent are selected. For example, alcohols such as methanol, ethanol, ethers such as ethyl ether, tetrahydrofuran, dioxane are preferably used. Among them, alcohols are most preferable. The solvent may be anhydrous, but hydrous solvent may sometimes provide a good result.

As such iodination agent, iodine, iodine-morpholine complex, iodine-sodium hypochlorite, potassium iodide-sodium hypochlorite, copper(I) iodide, zinc iodide and the like may be used. Iodine is most preferable. The amount of the iodination agent is suitably within the range from 1.0 to 1.5 mole, more preferably 1.0 to 1.1 mole per 1 mole of the compound [II]. This iodination reaction may be frequently facilitated using a base such as sodium hydroxide; potassium hydroxide; sodium hydride; sodium amide; potassium carbonate; tertiary amine, e.g. triethylaminde, pyridine; sodium methoxide; sodium ethoxide; n-butyllithium. As for the amount of the base, about 1.0 to 1.5 mole per 1 mole of the compound [II] is generally used. The reaction is carried out under cooling, at room temperature or under heating, but generally at room temperature. The reaction time varies depending on the types of the iodination agent and reaction temperature. Generally, it is from 5 minutes to 3 hours. After the reaction is over, the objective compound [I] can be isolated from the reaction mixture by employing conventional purification methods, for example, in a combination of solvent extraction, distillation, column chromatography, high performance liquid chromatography, recrystallization, etc.

As the starting compound [II], the commercially available ones or those produced by the known methods or analogous methods thereof can be used. The known methods for producing the starting compounds [II] include, for example, (i) condensation of propiolic acid and an amine [Japanese J. Pharmacology, 102 278 (1982); Japanese Patent Publication No. 58885/1985], (ii) reaction of a propioloyl halide or a propiolic anhydride with an amine, (iii) reaction of a propiolic acid ester with an amine [J. Org. Chem., 30 2660 (1965)], (iv) Ritter reaction of cyanoacetylene with an alcohol [J. Chem. Soc., (C) 406 - (1969)].

Further, the starting compounds [II] can be obtained using acrylamide as a starting material by processes shown in the following formula.

$$CH_2=CH-CONR_1R_2 + X_2 \rightarrow XCH_2-CHX-CONR_1R_2 \rightarrow$$

[A] [B]

$$CH_2=CX-CONR_1R_2 \rightarrow HC\equiv C-CONR_1R_2$$

[C] [II]

wherein X is Cℓ, Br or I

Therefore, to an acrylamide [A] is added a halogen such as chlorine, bromine, iodine to produce a dihalide [B], which is dehydrohalogenated to [II] by way of a 2-haloacrylamide [C]. Acrylamide [A] can be easily produced by the conventional methods using an acrylic acid ester, acryloyl chloride or acrylonitrile as a starting material.

Addition of halogen to acrylamide [A] is conducted by reacting equimolar of a halogen such as chlorine, bromine, iodine with one mole of [A] in an organic solvent which does not interfere with the reaction, such as chloroform, dichloromethane, ethyl ether, dioxane, benzene, toluene.

Dehydrohalogenation of dihalide [B] is completed by reacting [B] with a base in a solvent. The compound [II] can be obtained either by isolating the intermediate 2-haloacrylamide [C] to react [C] again with a base or by a single-step reaction of [B] without isolation of [C]. Solvents are not limited but an organic solvent such as chloroform, dichloromethane, ethyl ether, dioxane, benzene, toluene, acetonitrile, petroleum ether; water; or a mixed solvent of such organic solvent(s) and water is frequently used. As a base, sodium hydroxide; potassium hydroxide; sodium carbonate; potassium carbonate; sodium bicarbonate; potassium bicarbonate; sodium acetate; potassium acetate; ammonium acetate; a tertiary amine such as triethylamine, pyridine; potassium tert-butoxide; sodium hydride; sodium amide; sodium fluoride; potassium fluoride; or a basic ion exchange resin is used. In some cases, the reaction can be accelerated by adding a phase-transfer catalyst such as tetrabutylammonium bromide, tetraoctylammonium bromide, benzyltriethylammonium chloride, octadecyltrimethylammonium chloride, tetrabutylammonium hydrogensulfate in the reaction mixture.

$$(2) \quad I-C\equiv C-COOH + HNR_1R_2 \rightarrow I-C\equiv C-CO-NR_1R_2$$

[III] [IV] [I]

This is a process for production of the compound [I] of the present invention wherein 3-iodopropiolic acid [III], including a salt thereof, or halogenide thereof [III'], such as chloride, bromide, iodide, is reacted with amine [IV], including a salt thereof, for amidation. When iodopropiolic acid or a salt thereof [III] is used, the compound [III] is dissolved in an organic solvent, then reacted with an amine or a salt thereof [IV] to produce the compound [I]. As a salt of iodopropiolic acid, sodium salt, potassium salt, ammonium salt or the like may be used. On the other hand, as a salt of amine $HNR_1R_2$, a salt with inorganic acid such as hydrochloric acid, sulfuric acid; a salt with an organic acid such as acetic acid, paratoluenesulfonic acid may be used. As such organic solvent, ether such as ethyl ether, tetrahydrofuran; halogenated hydrocarbon such as dichloromethane, chloroform may be preferably used. The used organic solvent to be used may be anhydrous or hydrous. The amount of the amine [IV] is suitably in the range from 1.0 to 1.5 mole, more preferably in the range from 1.0 to 1.2 mole per 1 mole of the compound [III]. This amidation reaction may be frequently facilitated using dehydrating condensation agent such as N,N-dicyclohexylcarbodiimide, diethyl cyanophosphate, carbonyldiimidazole. The amount of the dehydrating condensation agent is generally about 1.0 to 1.5 mole per 1 mole of the compound [III]. The reaction is carried out at room temperature or under heating. Generally, the reaction is carried out at from 0 °C to room temperature. The reaction time is generally from 1 to 3 hours.

When iodopropioloyl halogenide [III'] is used, the compound [III'] is dissolved in an anhydrous organic solvent, then reacted with an amine [IV], including a salt thereof, to produce the compound [I]. As the salt of amine $HNR_1R_2$, those salts described above can be also used. As such organic solvent, halogenated hydrocarbon such as dichloromethane, chloroform; ether such as ethyl ether, tetrahydrofuran may be preferably used. The amount of the amine [IV] is suitably in the range from 1.0 to 1.5 mole, more preferably in the range from 1.0 to 1.2 mole per 1 mole of the compound [III']. This amidation reaction may be

frequently facilitated using a base such as pyridine or triethylamine. Based on 1 mole of the compound [III'], generally about 1.0 to 1.5 mole of the base may be used. The reaction is carried out under cooling, at room temperature or under heating. Generally, the reaction is carried out from -10 to 10 °C. The reaction time is generally from 1 to 3 hours.

When an acid or a salt thereof is used as the compound [III] or when the halogenide [III'] is used, the objective compound [I] can also be isolated from the reaction mixture by a combination of solvent extraction, distillation, column chromatography, high performance liquid chromatography, recrystallization or the like after the reaction is over. The starting compound [III] can be produced by the known processes [e.g., the processes described in Ann. Applied Biol., 36 250 (1949); Chem. Pharm. Bull., 14 1122 (1966)] or analogous process thereof. The halogenide [III'] can be produced from the compound [III] by the conventional methods using a halogenation agent.

$$(3)\quad \underset{[V]}{IXC{=}CY{-}CO{-}NR_1R_2} \quad \rightarrow \quad \underset{[I]}{I{-}C{\equiv}C{-}CO{-}NR_1R_2}$$

wherein X:I; Y:H or X:H; Y:Cl, Br, I

This is a process for production of the compound [I] of the present invention by dehydrohalogenation of 3,3-diiodo- or 2-halogeno-3-iodoacrylamide [V]. More particularly, this is a process for production of the compound [I] wherein the compound [V] is dissolved in a solvent, and reacted with a base. The solvent may not be particularly limited, but an organic solvent such as methanol, ethanol, acetonitrile, ethyl acetate; water alone or a mixed solvent of an organic solvent and water may be preferably used. As a base, sodium hydroxide; potassium hydroxide; sodium carbonate; potassium carbonate; sodium bicarbonate; potassium bicarbonate; tertiary amine such as triethylamine, pyridine may be used. The amount of the base is generally about 1.0 to 1.5 mole per 1 mole of the compound [V]. The reaction is carried out under cooling, at room temperature or under heating. The reaction time is generally from 1 to 5 hours. After reaction is over, the objective compound [I] can be isolated from the reaction mixture by a combination of solvent extraction, distillation, column chromatography, high performance liquid chromatography, recrystallization and the like. The compound [V] can be produced from dihalogenoacrylic acid or an ester thereof by the known processes [e.g., a process described in Japanese J. Pharmacology, 54 25 (1934)] or analogous process thereof.

## EFFECT OF THE INVENTION

The composition containing the compound [I] of the present invention which exhibits growth inhibiting activity against wide-variety of bacteria and fungi is useful as an antibacterial and antifungal agent. Particularly, the present composition is useful for prevention of damage caused by fungi in various field including industrial field since it exhibits strong antifungal activity. For example, it can be used for antideterioration agent for industrial products such as plastics, paint, resin or industrial water; a slime controlling agent; and further for a so-called wood preservative for prevention of against wood rot. Moreover, the compound [I] also exhibits antialgal, insecticidal, miticidal, anti-termite activities, and is a useful compound which can be widely available as a noxious organisms controlling agent. When the compound of the present invention is used as a noxious organisms controlling agent, it can be directly used without adding any other ingredients, but generally it may be mixed with a carrier, auxiliary agent and used in a dosage form such as emulsion, water dispersible powder, dusting powder, granule, oil borne agent or the like. Solid carrier used for dusting powder includes, for example, talc, clay, bentonite; solid carrier used for granule includes, for example, sepiolite, atapalgite, zeolite, natural pumice, perlite. The solvent used for an emulsion, oil borne agent or the like includes, for example, alcohols such as ethanol; ketones; glycol ethers; petroleum solvent; aliphatic hydrocarbons; aromatic hydrocarbons such as benzene; alkylbenzenes. As dispersion aid for water, for example, alkylbenzenesulfonate, higher alcohol sulfonate, polyoxyethylenealkylarylether, polyvinyl alcohol and the like may be used. In addition to the compound [I], another antibacterial and antifungal agent, antiseptic, insecticide or anti-termite may be mixed before use.

For example, when the compound [I] of the present invention is used as a wood preservative, it is generally used as an oil borne agent. The compound [I] is dissolved in the above solvent to the concentration of 0.1 to 10 % by weight, preferably to 0.5 to 5 % by weight. This oil borne agent is applied to wood with brush coating, spray coating or impregnation. In impregnation, for example, the amount of this oil borne agent containing 1 % by weight of the compound [I], required for treatment of 1 $m^3$ of wood is

generally 10 kg to 100 kg.

The compound [I] has high inhibiting activity against a wide variety of bacteria and fungi, as described above, as well as some advantageous properties such as low water-solubility, high thermal stability, resistance to self-discoloration, low toxicity to fish.

## EXAMPLE

The present invention will be further explained in detail in the following examples and reference examples.

### Reference example 1 N-(tert-Butyl)acrylamide

To a mixture of acrylonitrile (5.94 g), tert-butanol (8.00 g) and acetic acid (60ml), conc. sulfuric acid (10ml) was added under ice-cooling and the mixture was stirred for one hour. After further stirring for 3 days at room temperature, ice water (200 ml) was added to dilute the reaction mixture, and the precipitated crystals were filtered. The mother liquor was extracted with $CHC\ell_3$ and the extract was washed with water, aqueous $NaHCO_3$ solution and saturated aqueous $NaC\ell$ solution. After drying over $MgSO_4$, the solvent was removed under reduced pressure to yield colorless crystals. The crystals were combined and recrystallized from ethyl acetate to obtain N-(tert-butyl)acrylamide (11.28 g).
$^1$H-NMR ($CDC\ell_3$) $\delta$ 1.40 (s, 9H), 5.57 (dd, 1H, J = 10,2 Hz), 6.02 (dd, 1H, J = 17,10 Hz), 6.24 (dd, 1H, J = 17,2 Hz)

### Refernce example 2 N-(tert-Butyl)-2,3-dibromopropionamide

To a $CHC\ell_3$ solution (50 ml) of N-(tert-butyl)acrylamide (0.64 g), $Br_2$ (0.88 ml) was slowly added and the mixture was stirred for 4 hours at room temperature. Aqueous $Na_2S_2O_4$ solution was added to stop the reaction and the organic layer was separated. The organic layer was washed with water and saturated aqueous $NaC\ell$ solution. After drying over $MgSO_4$, the solvent was removed under reduced pressure to yield a colorless solid. Purification by silica gel column chromatography (eluant: ethyl acetate/n-hexane = 1:3) gave N-(tert-butyl)-2,3-dibromopropionamide (1.22 g).
$^1$H-NMR ($CDC\ell_3$) $\delta$ 1.39 (s, 9H), 3.81 (dd, 1H), 3.95 (dd, 1H), 4.34 (dd, 1H J = 17,2 Hz), 5.88 (s, 1H)

### Reference example 3 N-(tert-Butyl)-2-bromoacrylamide

To a solution of N-(tert-butyl)-2,3-dibromopropionamide (7.63 g) in ethyl alcohol (150 ml) was added sodium acetate (8.25 g) and hydroquinone (0.005 g), and the mixture was refluxed for 6 hours. After cooling the solution to room temperature and adding water to it until all of sodium acetate dissolved, ethyl alcohol was removed under reduced pressure. The residue was extracted with ether and the extract was washed with water and saturated aqueous $NaC\ell$ solution. After drying over $MgSO_4$, the solvent was removed under reduced pressure to obtain N-(tert-butyl)-2-bromoacrylamide (5.01 g).
$^1$H-NMR ($CDC\ell_3$) $\delta$ 1.41 (s, 9H), 5.97 (d, 1H), 6.95 (d, 1H)

### Reference example 4 N-(tert-Butyl)propiolamide (1)

To a solution of N-(tert-butyl)-2-bromoacrylamide (2.07 g) and tetrabutylammonium bromide (0.0322 g) dissolved in dioxane (20 ml) was added powdered potassium hydroxide (1.32 g) and the mixture was stirred for 4 hours at room temperature. After the completion of the reaction, the mixture was diluted with water and extracted with dichloromethane. The extract was washed with water and saturated aqueous $NaC\ell$ solution. After drying over $MgSO_4$, the solvent was removed to yield a solid. Purification by silica gel column chromatography (eluant: ethyl acetate/n-hexane = 1:5) gave N-(tert-butyl)propiolamide (0.62 g).
$^1$H-NMR ($CDC\ell_3$) $\delta$ 1.38 (s, 9H), 2.66 (s, 1H)

### Reference example 5 N-(tert-Butyl)propiolamide (2)

A mixture of N-(tert-butyl)-2-bromoacrylamide (1.00 g), tetrabutylammonium bromide (0.0167 g), Amberlyst A-26 (registerd name of ion exchange resin, 5 $cm^3$) and dioxane (10 ml) was stirred for 24 hours at room temperature. After the completion of the reaction, the resin was removed by filtration. The mixture was diluted with water and extracted with dichloromethane. The extract was washed with water and saturated

aqueous NaCℓ solution. After drying over $MgSO_4$, the solvent was removed. Purification by silica gel column chromatography (eluant: ethyl acetate/n-hexane = 1:5) gave N-(tert-butyl)propiolamide (0.24 g).

Reference example 6 N-(tert-Butyl)propiolamide (3)

To a hexamethylphosphoramide solution (5 ml) of N-(tert-butyl)-2-bromoacrylamide (0.41 g) was added sodium hydride (0.1 g) under ice-cooling, and the mixture was stirred for 2 hours at room temperature. Water was added to stop the reaction and the mixture was extracted with ethyl acetate. The extract was washed with water and saturated aqueous NaCℓ solution. After drying over $MgSO_4$, the solvent was removed. Purification by silica gel column chromatography (eluant: ethyl acetate/n-hexane = 1:5) gave N-(tert-butyl)propiolamide (0.12 g).

Reference example 7 N-(tert-Butyl)propiolamide (4)

A mixture of N-(tert-butyl)-2,3-dibromopropionamide (1.30 g), tetrabutylammonium bromide (0.05 g), powdered potassium hydroxide(0.60 g) and dioxane (10 ml) was stirred overnight at room temperature. The mixture was diluted with water and extracted with dichloromethane. The extract was washed with water and saturated aqueous NaCℓ solution. After drying over $MgSO_4$, the solvent was removed to yield a solid. Purification by silica gel column chromatography (eluant: ethyl acetate/n-hexane = 1:5) gave N-(tert-butyl)-propiolamide (0.22 g).

Example 1 N-(tert-Butyl)-3-iodopropiolamide

Sodium hydroxide (purity: 93 %, 1.80 g) was dissolved in methanol (80 ml), to which was added N-(tert-butyl)propiolamide (5.00 g). Subsequently, iodine (10.13 g) was added in small portions at room temperature with stirring. The reaction mixture was poured into 1 % aqueous solution of sodium thiosulfate (200 ml) and extracted three times with dichloromethane.

The extract was dried and concentrated and the residue was purified by column chromatography (silica gel, eluant: dichloromethane), and then by recrystallization from ethyl acetate/n-hexane (1:5) to yield N-(tert-butyl)-3-iodopropiolamide (7.75 g).
Melting Point: 142 - 143 °C
$^1$H-NMR (CDCℓ$_3$) δ 1.36 (s, 9H) 5.50 (br, 1H)
IR (KBr) cm$^{-1}$: 3,350, 2,170, 1,620

| Elemental Analysis (%) (for $C_7H_{10}NOI$) | | | |
|---|---|---|---|
| | C | H | N |
| Calcd. | 33.49 | 4.01 | 5.58 |
| Found | 33.76 | 4.01 | 5.54 |

Example 2 N-(n-Decyl)-3-iodopropiolamide

By a procedure analogous to Example 1, N-(n-decyl)-3-iodopropiolamide (1.38 g) was obtained from N-(n-decyl)propiolamide (1.32 g). As eluant for column chromatography and recrystallization solvent, ethyl acetate/n-hexane (1:4) and ethyl acetate/n-hexane (1:2) were used, respectively.
Melting Point: 105 - 106 °C
$^1$H-NMR (CDCℓ$_3$) δ 0.88 (t, 3H), 1.10 - 1.70 (m, 16H), 3.28 (m, 2H), 5.75 (br, 1H)
IR (KBr) cm$^{-1}$: 3,320, 2,170, 1,630, 1,610

| Elemental Analysis (%) (for $C_{13}H_{22}NOI$) | | | |
|---|---|---|---|
| | C | H | N |
| Calcd. | 46.58 | 6.61 | 4.18 |
| Found | 46.58 | 6.33 | 4.19 |

Example 3 N-(3-Chlorophenyl)-3-iodopropiolamide

By a procedure analogous to Example 1, N-(3-chlorophenyl)-3-iodopropiolamide (0.54 g) was obtained from N-(3-chlorophenyl)propiolamide (0.40 g). As eluant for column chromatography and recrystallization solvent, ethyl acetate/n-hexane (1:2) and ethyl acetate/n-hexane (1:5) were used, respectively.
Melting Point: 136.5 - 137.5 °C
$^1$H-NMR (CDCℓ$_3$) δ 7.02 - 7.68 (m, 4H)
IR (KBr) cm$^{-1}$: 3,230, 2,160, 1,640

| Elemental Analysis (%) (for $C_9H_5NOCℓI$) | | | |
|---|---|---|---|
| | C | H | N |
| Calcd. | 35.38 | 1.65 | 4.58 |
| Found | 35.38 | 1.69 | 4.41 |

Example 4 N-(n-Butyl)-3-iodopropiolamide

By a procedure analogous to Example 1, N-(n-butyl)-3-iodopropiolamide (2.98 g) was obtained from N-(n-butyl)propiolamide (2.99 g). As eluant for column chromatography and recrystallization solvent, ethyl acetate/n-hexane (1:3) and diethyl ether were used, respectively.
Melting Point: 109.5 - 111 °C
$^1$H-NMR (CDCℓ$_3$) δ 0.93 (t, 3H, J=7 Hz), 1.27 - 1.60 (m, 4H), 3.30 (dt, 2H, J=7, 6 Hz), 5.82 (br, 1H)
IR (KBr) cm$^{-1}$: 3,330, 2,160, 1,640

| Elemental Analysis (%) (for $C_7H_{10}NOI$) | | | |
|---|---|---|---|
| | C | H | N |
| Calcd. | 33.49 | 4.01 | 5.58 |
| Found | 33.53 | 3.90 | 5.58 |

Example 5 N-(sec-Butyl)-3-iodopropiolamide

By a proedure analogous to Example 1, N-(sec-butyl)-3-iodopropiolamide (0.46 g) was obtained from N-(sec-butyl)propiolamide (0.90 g). As eluant for column chromatography and recrystallization solvent, ethyl acetate/n-hexane (1:3) and dichloromethane were used, respectively.
Melting Point: 159 - 161 °C
$^1$H-NMR (CDCℓ$_3$) δ 0.92 (t, 3H, J=7 Hz), 1.15 (d, 3H, J=7 Hz), 1.49 (quint, 2H, J=7 Hz), 3.94 (tq, 1H, J=7, 7 Hz), 5.60 (br, 1H)

| Elemental Analysis (%) (for $C_7H_{10}NOI$) | | | |
|---|---|---|---|
| | C | H | N |
| Calcd. | 33.49 | 4.01 | 5.58 |
| Found | 33.59 | 3.78 | 5.61 |

Example 6 N-Isobutyl-3-iodopropiolamide

By a procedure analogous to Example 1, N-isobutyl-3-iodopropiolamide (0.80 g) was obtained from N-isobutylpropiolamide (3.19 g). As eluant for column chromatography and recrystallization solvent, ethyl acetate/n-hexane (1:3) and ethyl acetate/n-hexane were used, respectively.
Melting Point: 88 - 89 °C
$^1$H-NMR (CDCℓ$_3$) δ 0.93 (d, 6H, J=7 Hz), 1.81 (dqq, 1H, 7, 7, 7 Hz), 3.12 (t, 2H, J=7 Hz), 6.23 (br, 1H)

| Elemental Analysis (%) (for $C_7H_{10}NOI$) | | | |
|---|---|---|---|
| | C | H | N |
| Calcd. | 33.49 | 4.01 | 5.58 |
| Found | 33.19 | 3.84 | 5.57 |

Example 7 N-(n-Pentyl)-3-iodopropiolamide

By a procedure analogous to Example 1, N-(n-pentyl)-3-iodopropiolamide (3.40 g) was obtained from N-(n-pentyl)propiolamide (2.78 g). As eluant for column chromatography and recrystallization solvent, ethyl acetate/n-hexane (1:3) and ethyl acetate/n-hexane were used, respectively.
Melting Point: 96.5 - 98 °C
$^1$H-NMR ($CDCl_3$) δ 0.90 (t, 3H, J=7 Hz), 1.21 - 1.45 (m, 4H), 1.45 - 1.64 (m, 2H), 3.29 (dt, 2H, J=7, 7 Hz), 5.81 (br, 1H)

| Elemental Analysis (%) (for $C_8H_{12}NOI$) | | | |
|---|---|---|---|
| | C | H | N |
| Calcd. | 36.25 | 4.56 | 5.28 |
| Found | 36.25 | 4.53 | 5.06 |

Example 8 N-(n-Hexyl)-3-iodopropiolamide

By a procedure analogous to Example 1, N-(n-hexyl)-3-iodopropiolamide (3.81 g) was obtained from N-(n-hexyl) propiolamide (3.06 g). As eluant for column chromatography and recrystallization solvent, ethyl acetate/n-hexane (1:3) and ethyl acetate/n-hexane were used, respectively.
Melting Point: 97 - 98.5 °C
$^1$H-NMR ($CDCl_3$) δ 0.89 (t, 3H, J=7 Hz), 1.36 - 1.44 (m, 6H), 1.44 - 1.59 (m, 2H), 3.29 (dt, 2H, J=7, 7 Hz), 5.80 (br, 1H)

| Elemental Analysis (%) (for $C_9H_{14}NOI$) | | | |
|---|---|---|---|
| | C | H | N |
| Calcd. | 38.73 | 5.06 | 5.02 |
| Found | 38.97 | 5.08 | 5.23 |

Example 9 N-(n-Heptyl)-3-iodopropiolamide

By a procedure analogous to Example 1, N-(n-heptyl)-3-iodopropiolamide (2.72 g) was obtained from N-(n-heptyl)propiolamide (3.60 g). As eluant for column chromatography and recrystallization solvent, ethyl acetate/n-hexane (1:2) and ethyl acetate/n-hexane (1:3) were used, respectively.
Melting Point: 108 - 110 °C
$^1$H-NMR ($CDCl3$) δ 0.88 (t, 3H, J=7 Hz), 1.22 - 1.39 (m, 8H), 1.39 - 1.62 (m, 2H), 3.28 (dt, 2H, J=7, 7 Hz), 5.79 (br, 1H)

| Elemental Analysis (%) (for $C_{10}H_{16}NOI$) | | | |
|---|---|---|---|
| | C | H | N |
| Calcd. | 40.97 | 5.50 | 4.78 |
| Found | 41.09 | 5.47 | 4.84 |

Example 10 N-(n-Octyl)-3-iodopropiolamide

By a procedure analogous to Example 1, N-(n-octyl)-3-iodopropiolamide (4.37 g) was obtained from N-(n-octyl)propiolamide (3.63 g). As eluant for column chromatography and recrystallization solvent, ethyl acetate/n-hexane (1:3) and ethyl acetate/n-hexane were used, respectively.
Melting Point: 101 - 102 °C
$^1$H-NMR ($CDC\ell_3$) δ 0.89 (t, 3H, J = 7 Hz), 1.27 (br, 10H), 1.43 - 1.63 (m, 2H), 3.28 (dt, 2H, J = 7, 7 Hz), 5.82 (br, 1H)

| Elemental Analysis (%) (for $C_{11}H_{18}NOI$) | | | |
|---|---|---|---|
| | C | H | N |
| Calcd. | 43.01 | 5.91 | 4.56 |
| Found | 42.70 | 5.94 | 4.36 |

Example 11 N-(n-Nonyl)-3-iodopropiolamide

By a procedure analogous to Example 1, N-(n-nonyl)-3-iodopropiolamide (3.33 g) was obtained from N-(n-nonyl)propiolamide (3.69 g). As eluant for column chromatography and recrystallization solvent, ethyl acetate/n-hexane (1:3) and dichloromethane were used, respectively.
Melting Point: 107 - 107.5 °C
$^1$H-NMR ($CDC\ell_3$) δ 0.88 (t, 3H, J = 7 Hz), 1.27 (br, 12H), 1.43 - 1.61 (m, 2H) 3.28 (dt, 2H, J = 7, 7 Hz), 5.82 (br, 1H)

| Elemental Analysis (%) (for $C_{12}H_{20}NOI$) | | | |
|---|---|---|---|
| | C | H | N |
| Calcd. | 44.87 | 6.28 | 4.36 |
| Found | 44.83 | 6.17 | 4.40 |

Example 12 N-(n-Undecyl)-3-iodopropiolamide

By a procedure analogous to Example 1, N-(n-undecyl)-3-iodopropiolamide (3.07 g) was obtained from N-(n-undecyl)propiolamide (3.16 g). As eluant for column chromatography and recrystallization solvent, ethyl acetate/n-hexane (1:3) and ethyl acetate/n-hexane were used, respectively.
Melting Point: 109.5 - 110.5 °C
$^1$H-NMR ($CDC\ell_3$) δ 0.88 (t, 3H, J = 7 Hz), 1.26 (br, 16H), 1.43 - 1.60 (m, 2H), 3.28 (dt, 2H, J = 7, 7 Hz), 5.77 (br, 1H)

| Elemental Analysis (%) (for $C_{14}H_{24}NOI$) | | | |
|---|---|---|---|
| | C | H | N |
| Calcd. | 48.15 | 6.93 | 4.01 |
| Found | 48.22 | 7.12 | 4.03 |

Example 13 N-(n-Dodecyl)-3-iodopropiolamide

By a procedure analogous to Example 1, N-(n-dodecyl)-3-iodopropiolamide (3.09 g) was obtained from N-(n-dodecyl)propiolamide (3.00 g). As eluant for column chromatography and recrystallization solvent, dichloromethane and ethyl acetate/n-hexane (1:3) were used, respectively.
Melting Point: 102.5 - 104 °C
$^1$H-NMR ($CDC\ell_3$) δ 0.88 (t, 3H, J = 7 Hz), 1.26 (br, 18H), 1.42 - 1.61 (m, 2H), 3.29 (dt, 2H, J = 7, 7 Hz), 5.78

(br, 1H)

| Elemental Analysis (%) (for $C_{15}H_{26}NOI$) | | | |
|---|---|---|---|
| | C | H | N |
| Calcd. | 49.59 | 7.21 | 3.86 |
| Found | 49.76 | 7.21 | 3.83 |

Example 14 N-(n-Tetradecyl)-3-iodopropiolamide

By a procedure analogous to Example 1, N-(n-tetradecyl)-3-iodopropiolamide (2.05 g) was obtained from N-(n-tetradecyl)propiolamide (2.60 g). As eluant for column chromatography and recrystallization solvent, ethyl acetate/n-hexane (1:2) and ethyl acetate/n-hexane were used, respectively.
Melting Point: 104 - 105 °C
$^1$H-NMR ($CDCl_3$) δ 0.88 (t, 3H, J = 7 Hz), 1.26 (br, 22H), 1.43 - 1.60 (m, 2H), 3.27 (dt, 2H, J = 7, 7 Hz), 5.79 (br, 1H)

| Elemental Analysis (%) (for $C_{17}H_{30}NOI$) | | | |
|---|---|---|---|
| | C | H | N |
| Calcd. | 52.18 | 7.73 | 3.58 |
| Found | 52.34 | 7.60 | 3.49 |

Example 15 N-(n-Octadecyl)-3-iodopropiolamide

By a procedure analogous to Example 1, N-(n-octadecyl)-3-iodopropiolamide (0.96 g) was obtained from N-(n-octadecyl)propiolamide (1.13 g). As eluant for column chromatography and recrystallization solvent, ethyl acetate/n-hexane (1:2) and ethyl acetate/n-hexane were used, respectively.
Melting Point: 112 - 113 °C
$^1$H-NMR ($CDCl_3$) δ 0.88 (t, 3H, J = 7 Hz), 1.18 - 1.36 (br, 30H), 1.42 - 1.61 (m, 2H), 3.28 (dt, 2H, J = 7, 7 Hz)

| Elemental Analysis (%) (for $C_{21}H_{38}NOI$) | | | |
|---|---|---|---|
| | C | H | N |
| Calcd. | 56.37 | 8.56 | 3.13 |
| Found | 56.31 | 8.38 | 3.08 |

Example 16 N-(2-Phenylethyl)-3-iodopropiolamide

By a procedure analogous to Example 1, N-(2-phenylethyl)-3-iodopropiolamide (1.75 g) was obtained from N-(2-phenylethyl)propiolamide (1.63 g). As eluant for column chromatography, ethyl acetate/n-hexane (1:3) was used. An oily product was obtained.
$^1$H-NMR ($CDCl_3$) δ 2.84 (t, 2H, J = 7 Hz), 3.57 (dt, 2H, J = 7, 7 Hz), 5.80 (br, 1H), 7.16 - 7.39 (m, 5H)

| Elemental Analysis (%) (for $C_{11}H_{10}NOI$) | | | |
|---|---|---|---|
| | C | H | N |
| Calcd. | 44.17 | 3.37 | 4.68 |
| Found | 44.12 | 3.35 | 4.66 |

Example 17 N-(3-Fluorophenyl)-3-iodopropiolamide

By a procedure analogous to Example 1, N-(3-fluorophenyl)-3-iodopropiolamide (1.84 g) was obtained from N-(3-fluorophenyl)propiolamide (1.36 g). As eluant for column chromatography and recrystallization solvent, ethyl acetate/n-hexane (1:10) and ethyl acetate/n-hexane (1:5) were used, respectively.
Melting Point: 90 - 91.3 °C
$^1$H-NMR ($CDCl_3$) δ 6.80 - 6.91 (m, 1H), 7.10 - 7.18 (m, 1H), 7.23 - 7.35 (m, 1H), 7.42 - 7.51 (m, 1H)

| Elemental Analysis (%) (for $C_9H_5NOFI$) | | | |
|---|---|---|---|
| | C | H | N |
| Calcd. | 37.40 | 1.74 | 4.85 |
| Found | 37.30 | 1.74 | 4.83 |

Example 18 N,N-Di(n-butyl)-3-iodopropiolamide

By a procedure analogous to Example 1, N,N-di(n-butyl)-3-iodopropiolamide (1.63 g) was obtained from N,N-di(n-butyl)propiolamide (1.41 g). As eluant for column chromatography, ethyl acetate/n-hexane (1:3) was used.
Oily.
$^1$H-NMR ($CDCl_3$) δ 0.88 - 1.00 (m, 6H), 1.21 - 1.68 (m, 8H), 3.30 - 3.38 (m, 2H), 3.47 - 3.58 (m, 2H)

Example 19 1-(3-Iodopropioloyl)piperidine

By a procedure analogous to Example 1, 1-(3-iodopropioloyl)piperidine (0.75 g) was oabtained from 1-propioloylpiperidine (1.37 g). As eluant for column chromatography and recrystallization solvent, ethyl acetate/n-hexane (1:3) and ethyl acetate/n-hexane were used, respectively.
Melting Point: 124.5 - 125 °C
$^1$H-NMR ($CDCl_3$) δ 1.52 - 1.70 (m, 6H), 3.53 - 3.60 (m, 2H), 3.66 - 3.73 (m, 2H)

| Elemental Analysis (%) (for $C_8H_{10}NOI$) | | | |
|---|---|---|---|
| | C | H | N |
| Calcd. | 36.52 | 3.83 | 5.32 |
| Found | 36.44 | 3.81 | 5.23 |

Example 20 4-(3-Iodopropioloyl)-3,5-dimethylmorpholine

By a procedure analogous to Example 1, 4-(3-iodopropioloyl)-3,5-dimethylmorpholine (0.51 g) was obtained from 4-propioloyl-3,5-dimethylmorpholine (1.17 g). As eluant for column chromatography and recrystallization solvent, ethyl acetate/n-hexane (1:3) and ethyl acetate/n-hexane were used, respectively.
Melting Point: 124.6 - 126 °C
$^1$H-NMR ($CDCl_3$) δ 1.22 (t, 6H, J=7 Hz), 2.43 (dd, 1H, J=13, 11 Hz), 2.86 (dd 1H, J=13, 11 Hz), 3.43 - 3.68 (m, 2H), 4.12 (dt, 1H, J=13, 2 Hz), 4.36 (dt, 1H, J=13, 2 Hz)

| Elemental Analysis (%) (for $C_9H_{12}NO_2I$) | | | |
|---|---|---|---|
| | C | H | N |
| Calcd. | 36.88 | 4.13 | 4.78 |
| Found | 36.91 | 4.12 | 4.65 |

Example 21 1-(3-Iodopropioloyl)-4-phenylpiperazine

By a procedure analogous to Example 1, 1-(3-iodopropioloyl)-4-phenylpiperazine (1.78 g) was obtained from 1-propioloyl-4-phenylpiperazine (1.49 g). As eluant for column chromatography and recrystallization solvent, ethyl acetate/n-hexane (1:2) and ethyl acetate were used, respectively.
Melting Point: 157.5 - 159 °C
$^1$H-NMR ($CDCl_3$) δ 3.12 - 3.27 (m, 4H), 3.76 - 3.83 (m, 2H), 3.88 - 3.95 (m, 2H), 6.88 - 6.97 (m, 3H), 7.24 - 7.34 (m, 2H)

| Elemental Analysis (%) (for $C_{13}H_{13}N_2OI$) | | | |
|---|---|---|---|
| | C | H | N |
| Calcd. | 45.90 | 3.85 | 8.24 |
| Found | 45.74 | 3.63 | 7.99 |

Example 22 N-Cyclohexyl-3-iodopropiolamide

By a procedure analogous to Example 1, N-cyclohexyl-3-iodopropiolamide (0.44 g) was obtained from N-cyclohexylpropiolamide (0.56 g). As eluant for column chromatography and recrystallization solvent, ethyl acetate/n-hexane (1:2) and ethyl acetate/n-hexane were used, respectively.
Melting Point: 124 - 126 °C
$^1$H-NMR ($CDCl_3$) δ 1.05 - 2.02 (m, 10H), 3.71 - 3.90 (m, 1H), 5.69 (1H, br)

Example 23 N-Allyl-3-iodopropiolamide

By a procedure analogous to Example 1, N-allyl-3-iodopropiolamide (0.63 g) was obtained from N-allylpropiolamide (2.18 g). As eluant for column chromatography and recrystallization solvent, ethyl acetate/n-hexane (1:9) and ethyl ether/n-hexane were used, respectively.
Melting Point: 69 - 69.5 °C
$^1$H-NMR ($CDCl_3$) δ 3.88 - 3.94 (m, 2H), 5.15 - 5.29 (m, 2H), 5.73 - 5.92 (m, 2H)

| Elemental Analysis (%) (for $C_6H_6NOI$) | | | |
|---|---|---|---|
| | C | H | N |
| Calcd. | 30.66 | 2.57 | 5.96 |
| Found | 30.85 | 2.68 | 5.95 |

Example 24 N-(4-Chlorophenyl)-3-iodopropiolamide

By a procedure analogous to Example 1, N-(4-chlorophenyl)-3-iodopropiolamide (1.20 g) was obtained from N-(4-chlorophenyl)propiolamide (2.50 g). As eluant for column chromatography and recrystallization solvent, ethyl acetate/n-hexane (1:9) and ethyl acetate/n-hexane were used, respectively.
Melting Point: 125 - 126 °C
$^1$H-NMR ($CDCl_3$) δ 7.24 - 7.35 (m, 2H), 7.4 - 7.58 (d, 2H),

| Elemental Analysis (%) (for $C_9H_5NOClI$) | | | |
|---|---|---|---|
| | C | H | N |
| Calcd. | 35.38 | 1.65 | 4.58 |
| Found | 35.46 | 1.61 | 4.54 |

Example 25 N-(2,4-Dichlorophenyl)-3-iodopropiolamide

By a procedure analogous to Example 1, N-(2,4-dichlorophenyl)-3-iodopropiolamide (0.62 g) was obtained from N-(2,4-dichlorophenyl)propiolamide (2.54 g). As eluant for column chromatography and recrystallisation solvent, ethyl acetate/n-hexane (1:7) and ethyl acetate/n-hexane were used, respectively.
Melting Point: 129.5 - 131 °C
$^1$H-NMR (CDCℓ$_3$) δ 7.26 (m, 1H), 7.41 (d, 1H, J = 2 Hz), 7.83 (m, 1H), 8.29 (d, 1H, J = 9 Hz)

Example 26 N-(3-Trifluoromethylphenyl)-3-iodopropiolamide

By a procedure analogous to Example 1, N-(3-trifluoromethylphenyl)-3-iodopropiolamide (1.15 g) was obtained from N-(3-trifluoromethylphenyl)propiolamide (3.46 g). As eluant for column chromatography and recrystallization solvent, ethyl acetate/n-hexane (1:8) and ethyl acetate/n-hexane were used, respectively.
Melting Point: 94 - 95.5 °C
$^1$H-NMR (CDCℓ$_3$) δ 7.35-7.55 (m, 2H), 7.55-7.85 (m, 2H)

| Elemental Analysis (%) (for $C_{10}H_5NOF_3I$) | | | |
|---|---|---|---|
| | C | H | N |
| Calcd. | 35.42 | 1.49 | 4.13 |
| Found | 35.31 | 1.62 | 4.37 |

Example 27 N-Phenyl-3-iodopropiolamide

By a procedure analogous to Example 1, N-phenyl-3-iodopropiolamide (3.87 g) was obtained from N-phenylpropiolamide (10.73 g). As eluant for column chromatography and recrystallization solvent, ethyl acetate/n-hexane (1:5) and ethyl acetate/n-hexane were used, respectively.
Melting Point: 139 - 141 °C
$^1$H-NMR (CDCℓ$_3$) δ 7.15 (t, 1H), 7.34 (t, 2H), 7.58 (d, 2H)

Example 28 N-(3-Nitrophenyl)-3-iodopropiolamide

By a procedure analogous to Example 1, N-(3-nitrophenyl)-3-iodopropiolamide (0.15 g) was obtained from N-(3-nitrophenyl)propiolamide (0.19 g). As eluant for column chromatography and recrystallization solvent, ethyl acetate/n-hexane (1:4) and ethyl acetate/n-hexane were used, respectively.
Melting Point: 183.5 - 185 °C
$^1$H-NMR (CDCℓ$_3$) δ 7.55 (m, 1H), 7.97 (m, 2H), 8.35 (m, 1H)

| Elemental Analysis (%) (for $C_9H_5N_2O_3I$) | | | |
|---|---|---|---|
| | C | H | N |
| Calcd. | 34.20 | 1.59 | 8.86 |
| Found | 34.35 | 1.73 | 8.79 |

Example 29 N-(2-Thienylmethyl)-3-iodopropiolamide

By a procedure analogous to Example 1, N-(2-thienylmethyl)-3-iodopropiolamide(1.02 g) was obtained from N-(2-thienylmethyl)propiolamide (0.98 g). As eluant for column chromatography and recrystallization solvent, ethyl acetate/n-hexane (1:9) and dichloromethane/n-hexane were used, respectively.
Melting Point: 115 - 117 °C
$^1$H-NMR (CDCℓ$_3$) δ 4.64(d, 2H, J = 5.8Hz), 6.94-7.15 (m, 2H), 7.24-7.27 (m, 1H)

TEST EXAMPLE

Test examples are shown hereinafter to demonstrate availability of the compound of the present invention.

Test example 1 Antibacterial and antifungal activity

Antibacterial and antifungal activity tests for various bacteria and fungi were conducted. The tests were carried out by the conventional paper disc method. That is, a paper disc (d = 8 mm) was dipped in aqueous or methanol solution of the compound of the present invention (concentration : 1,000 μg/ml), then dried and used.

As bacteria to be tested,
① Staphylococcus aureus IFO-12732 [hereinafter abbreviated to Sta.]
② Bacillus subtilis IFO-13719 [Bac.]
③ Escherichia coli IFO-3301 [Esc.]
were used. As yeast to be tested,
④ Saccharomyces cerevisiae IFO-0210 [Sac.]
⑤ Candida albicans IFO-0583 [Can.]
were used. As fungi to be tested,
⑥ Aspergillus niger IFO-4407 [Asp.]
⑦ Penicillium citrinum IFO-7784 [Pen.]
⑧ Tyromyces palustris IFO-30339 [Tyr.]
⑨ Coriolus versicolor IFO-30340 [Cor.]
were used. Among the above fungi, Tyr. and Cor. are wood rot fungi.

As the test media, agar media shown below were used. That is, TSA (Trypticase Soy Agar) medium was used for Sta., Bac. and Esc. ; YNB (Yeast Nitrogen Base) agar medium was used for Sac. and Can.; SDA (Sabouraud Dextrose Agar) medium was used for Asp. and Pen.; and PDA (Potato Dextrose Agar) medium was used for Try. and Cor.

The determination of antibacterial and antifungal effectiveness was carried out by measuring the diameter of the growth inhibition circle generated on the circumference of the paper disc. The results are shown in the following Table 1. The indication of effectiveness is given as follows:

-: no growth inhibition circle was observed.
+: diameter of the growth inhibition circle was 9 - 20 mm
+ +: diameter of the growth inhibition circle was 21 - 40 mm
+ + +: diameter of the growth inhibition circle was 41 mm or more

【Table 1】

| Test Bacteria | Compound (Example No.) 1 | 2 | 3 | 4 | 5 | 6 | 7 | 8 |
|---|---|---|---|---|---|---|---|---|
| Sta. (IFO-12732) | + | + | ++ | ++ | ++ | ++ | ++ | ++ |
| Bac. (IFO-13719) | + | + | ++ | + | + | ++ | + | + |
| Esc. (IFO-3301) | + | - | + | + | + | - | + | + |
| Sac. (IFO-0210) | ++ | + | ++ | ++ | ++ | ++ | ++ | ++ |
| Can. (IFO-0583) | ++ | + | ++ | ++ | ++ | ++ | ++ | ++ |
| Asp. (IFO-4407) | ++ | + | ++ | ++ | ++ | ++ | ++ | ++ |
| Pen. (IFO-7784) | +++ | + | +++ | +++ | +++ | ++ | +++ | +++ |
| Tyr. (IFO-30339) | +++ | + | +++ | +++ | +++ | +++ | +++ | +++ |
| Cor. (IFO-30340) | +++ | ++ | +++ | +++ | +++ | ++ | +++ | +++ |

| Test Bacteria | Compound (Example No.) 9 | 10 | 11 | 16 | 17 | 18 | 19 | 20 |
|---|---|---|---|---|---|---|---|---|
| Sta. (IFO-12732) | + | + | + | ++ | ++ | ++ | ++ | ++ |
| Bac. (IFO-13719) | + | - | - | ++ | ++ | + | ++ | + |
| Esc. (IFO-3301) | - | - | - | + | + | - | + | - |
| Sac. (IFO-0210) | ++ | ++ | + | +++ | ++ | ++ | ++ | ++ |
| Can. (IFO-0583) | ++ | ++ | + | ++ | ++ | ++ | ++ | + |
| Asp. (IFO-4407) | ++ | + | + | ++ | ++ | ++ | ++ | ++ |
| Pen. (IFO-7784) | +++ | ++ | ++ | +++ | +++ | +++ | +++ | +++ |
| Tyr. (IFO-30339) | +++ | ++ | ++ | +++ | +++ | +++ | +++ | +++ |
| Cor. (IFO-30340) | +++ | +++ | +++ | +++ | +++ | +++ | +++ | +++ |

| Test Bacteria | Compound (Example No.) 21 | 22 | 23 | 24 | 25 | 26 |
|---|---|---|---|---|---|---|
| Sta. (IFO-12732) | ++ | ++ | ++ | ++ | ++ | ++ |
| Bac. (IFO-13719) | + | + | + | + | + | + |
| Esc. (IFO-3301) | - | + | + | + | + | + |
| Sac. (IFO-0210) | ++ | ++ | ++ | ++ | ++ | ++ |
| Can. (IFO-0583) | ++ | ++ | ++ | ++ | ++ | ++ |
| Asp. (IFO-4407) | ++ | ++ | ++ | ++ | ++ | ++ |
| Pen. (IFO-7784) | +++ | +++ | +++ | +++ | +++ | +++ |
| Tyr. (IFO-30339) | +++ | +++ | +++ | +++ | +++ | ++ |
| Cor. (IFO-30340) | +++ | +++ | +++ | +++ | +++ | +++ |

Test example 2 Preserving activity for wood preservatives

This test was conducted according to Specification No. 1 of Japan Wood Preserving Association, enacted in 1979, amended in 1989 and titled "Test methods of preserving activity for wood preservatives used in the coating, spraying and dipping treatment".

2-1 Fungi to be used

① Tyromyces palustris (Ber. et Curt.) Murr., FFPRI 0507 --"Ouzuratake"
② Coriolus versicolor(L. ex Fr.) Quélet, FFPRI 1030 --"Kawaratake"
These two fungi are classified as wood rot fungi.

2-2 Culture medium

Culture media comprised culture jars (cylindrical jars of bottom diameter 8-9 cm, opening diameter 6-7 cm and height 15-18 cm), in which quartz sand of 250 g (the size of which corresponds to 20-30 mesh) was put first, and then the solution of 70 ml containing 2.0 % malt extracts and 1.0 % peptone was introduced, over which chips of the beech or other broadleaf tree were spread at about 2 mm thickness, and then sterilized at 120 °C for 30 minutes in the wet heated sterilizer.

2-3 Method for culture

Fungi were cultured at 26 ± 2 °C with shaking using the culture solution of 2.0 % malt extracts and 1.0 % peptone. After sufficient breeding was obtained, the fungi solution of about 3 ml was spread all over the culture medium, and then the fungi were cultured at 26 ± 2 °C with the relative humidity of over 70 %. After 10-15 days when the fungi layer sufficiently covered the culture medium, the fungi were served for the tests.

2-4 Wood specimens

Wood specimens were the sapwood of intact cedar and beech, the dimension of which was 5 mm thick, 20 mm wide and 40 mm long. The grain face was of 20 mm width and 40 mm length. The side faces were treated with cold-setting epoxy resin.

2-5 Tree types versus fungi types

The antimicrobial operation was performed with specifying types of fungi for corresponding type of trees. The combinations were as follows:
Cedar----Tyromyces palustris ("Ouzuratake")
Beech----Coriolus versicolor ("Kawaratake")

2-6 Test specimens

The test specimens were of two types, treated and untreated specimens, respectively.

2-6-1 Treated specimens

The treated specimens referred to the wood specimens coated by brushing, spraying or dipping with wood preservatives. The amount of applied preservatives was 110 ± 10 g/m$^2$.

2-6-2 The amount of applied preservatives

The amount of applied preservatives for each test specimen was calculated by the following equation:
The amount of applied preservatives (g/m$^2$) = ($W_1$-$W_2$)/A
wherein
$W_1$ :weight of the test specimen after absorption (g),
$W_2$ : weight of the test specimen before absorption (g)
and
A: surface area of test specimen except both butt surfaces (m$^2$)

2-7 Tests

2-7-1 The test specimens prepared in the previous section were dried at 60 ± 2 °C with a recirculating dryer for 48 hours, and were allowed to stand in a desiccator for about 30 minutes, and then the weight ($W_3$) was measured to an accuracy of 0.01 g.

2-7-2 Three of each treated and untreated specimens were assembled in the Teflon casing. After sterilizing, the two assemblies were put on their 40 × 5 mm face on the fungi layer in the separate jar. They were kept at 26 ± 2 °C under the relative humidity of over 70 % for eight weeks.

2-7-3 After the eight weeks had elapsed, the test specimens were taken out, and mycelia and other attachments of them were removed neatly. After air-drying for 24 hours, the test specimens were dried at

60 ± 2 °C in a recirculating dryer for 48 hours, and were allowed to stand in a desiccator for about 30 minutes, and then the weight ($W_4$) was measured to an accuracy of 0.01 g.

2-8 Test results

The test results were presented by the ratio of average weight loss of test specimens due to antimicrobial operation. The standard deviation and coefficient of variation were also determined. The ratio of weight loss for each test specimen was calculated by the following equation, and its average was also determined: The ratio of weight loss(%) = $(W_3 - W_4) / W_3 \times 100$. The test results are shown below in Table 2. Lower value of the ratio of weight loss corresponds to higher wood preserving activity. As demonstrated in Table 2, example compounds Nos. 1, 2, 4, 5, 6, 7 and 8 show excellent wood preserving activity.

Table 2

| Ratio of weight loss (%) | | | | | | | | | | | | |
|---|---|---|---|---|---|---|---|---|---|---|---|---|
| Fungi | Compound (Example No.) | | | | | | | | | | | |
| | 1 | 2 | 4 | 5 | 6 | 7 | 8 | 9 | 10 | 11 | R | $N_1$ | $N_2$ |
| T. palustris | 2.4 | 0.2 | 0.0 | 2.5 | 0.0 | 2.2 | 1.3 | 3.3 | 5.0 | 5.8 | 1.3 | 28.9 | 23.3 |
| C. versicolor | 11.9 | 15.9 | 1.6 | 7.2 | 1.5 | 3.1 | 1.7 | 12.0 | 8.4 | 14.2 | 6.4 | 16.1 | 17.8 |

Note. Compounds 1-11 : 0.5% xylene solution
R : Reference compound- $I-C{\equiv}CCH_2O-CO-NH(CH_2)_3CH_3$. 0.5% xylene solution
$N_1$ : untreated
(untreated in the case of tests for example compounds 2,4 and 6)
$N_2$ : untreated
(untreated in the case of tests for example compounds 1, 5, 7, 8, 9, 10 and 11 and R)

Test example 3 Fungicide activity for wood fungicide

This test was conducted according to Specification No.2 of Japan Wood Preserving Association, enacted in 1979 and titled "Test methods of fungicide activity for wood fungicide".

3-1 The fungi to be used

① Aspergillus niger van Tieghem IFO 6341 = ATCC 6275
② Penicillium funiculosum Thom (formerly : Penicillium luteum Zukal) IFO 6345 = ATCC 9644 .
③ Rhizopus javanicus Takeda (formerly : Rhizopus nigricans Ehrenberg S.N. 32) IFO 6354
④ Aureobasidium pullulans (de Bary) Arnaud IFO 6353 = IAM (H. Iizuka, F-24)
⑤ Gliocladium virens Miller, Gidden & Fosteb IFO 6355 = ATCC 9645 (formerly : Trichoderma viride, T.I.)

The fungi ① , ② , ③ and ⑤ are classified as fungi imperfecti, and the fungus ④ as blue stain fungus.

3-2 Culture medium

The Culture medium comprised the potato-sap agar culture medium (specified in the Japan Industrial Standard (JIS) Z 2911- 1960) of 50 ml, put in the conical flask of 100 ml, sterilized by steam at 1.4-1.6 $kgf/cm^2$ .G (137.2-156.8 KPa) for 30 minutes.

3-3 Method for culture

The fungi to be used were inoculated into the culture medium specified in the previous section and were cultured at 26 ± 2 °C under the relative humidity of 70-80 % for two weeks.

3-4 Preparing spore suspension

The potato sap of 50 ml, sterilized, cooled to room temperature and to which dioctyl sodium sulfosuccinate, a wetting agent, was added so as to become 0.005 %, was dropped to the fully breeding fungi layer, and was agitated with a platinum spatula in such a way that the spore was torn off. The resulting liquid filtered by sterilized gauze was the spore suspension.

3-5 Preparing Petri dish

3-5-1 A Petri dish measured 90-100 mm in diameter and 20 mm in height.
3-5-2 Agar liquid (which has no nutrients) of 2 % sterilized by steam was dropped onto the Petri dish sterilized by dry heat, and then was solidified.
3-5-3 Three mm diameter sterilized glass bars or plastic nets made of polypropylene, etc. were placed to prevent the test specimens from touching with agar directly.

3-6 Wood specimens

3-6-1 The type of trees for wood specimens was beech. The cross section was of grain face, and the dimension was 20 mm wide 3 mm thick and 50 mm long.
3-6-2 Wood specimens were supplied with nutrients in such a way that they were dipped into the potato-sap (the one in the section 3-2, but agar was not added) for 3 minutes.
3-6-3 Nutrient-supplied wood specimens, after drying at 60 °C, were used for testing immediately. They were temporarily stored at 0 °C, but they were served to tests as soon as possible.

3-7 Test specimens

3-7-1 Types of test specimens

The test specimens were of two types, treated and untreated speciments. The treated specimens referred to the one treated with fungicide. The untreated specimens were of two types, the one really untreated and the other treated with solvent only.

3-7-2 Treated test specimens

3-7-2-1 Nutrients-supplied wood specimens were dried at 60 ± 2 °C, and then their weights ($W_0$) were measured. Test specimens of 10-20 pieces were piled up in a one liter beaker in such a way that two specimens constant space apart from each other were alternately put on the other two specimens also constant space apart from each other. On the top of the two specimens, a weight was placed. Through the test specimens thus arranged, the fungicide solution was introduced. The level of the fungicide solution was one centimeter above the top specimens. The test specimens were dipped for three minutes after the fungicide solution introduction.

After finishing dipping, the test specimens were wiped softly, and then the weights ($W_1$) were measured immediately. As control experiment, the same procedure as the above was done using solvent without fungicide.

3-7-2-2 The amounts of solution pick-up and fungicide pick-up were calculated with the following equations:

The amount of solution absorption: $R_L = W_1 - W_0$

The amount of fungicide absorption: $R_C = R_L \times$ fungicide concentration

3-7-2-3 Wood specimens treated were placed without contacting each other, then were air-dried for two days, and then served as the treated test specimens.

3-7-3 Preparing test specimens prior to tests

The test specimens were put into one liter beakers separately according to the concentration of fungicide solution used for treating, then were dipped for three minutes in the added sterilizing water, and then water was removed. The untreated test specimens were also subject to the same treatment.

3-8 Tests

3-8-1 Placing test specimens

Three test specimens which were subject to the same treatment were placed in a Petri dish in parallel with each other without contacting each other. The test specimens were placed on their faces 2 mm wide.

3-8-2 Inoculation of fungi

The surfaces of the three test specimens in the Petri dish were coated with the single spore suspension of 2 ml using a brush.

3-8-3 Culturing

Culturing was carried out at 26 × 2 °C under the relative humidity of 70-80 % for 4 weeks. If the rating of test specimens reached 3, the elapsed days were recorded.

3-9 Test results

3-9-1 Rating

The growth of fungi was observed for each type of fungi, for each concentration of fungicide solution and for each specimen after 4 weeks. The rating was determined according to the list of rating shown in the following.

| Rating | Growth of fungi |
|---|---|
| 0 | No fungi growth is observed on the surfaces of the test specimen |
| 1 | Fungi growth is observed only on the side surfaces |
| 2 | Fungi growth is observed on the top surface, but the area is less than one third. |
| 3 | Fungi growth is observed on the top surface, and the area is more than one third. |

3-9-2 Average rating

The average rating (A) was determined for each type of fungi with the following equation:

Average rating (A) $= (a_1 + a_2 + a_3 + a_4 + a_5 + a_6) / 6$

wherein $a_1$, $a_2$ ...$a_6$ : rating for each test specimen.

3-9-3 Damage value

The damage value was determined with the following equation after determining the sum of average rating by adding each rating of test specimens for each concentration of fungicide solution:

$S = A_1 + A_2 + A_3 + A_4 + A_5$

wherein $A_1$, $A_2$.., $A_5$: average rating for each type of fungi

$D = S_1 / S_0 \times 100$

wherein
$S_0$ : S for untreated test specimens
$S_1$ : S for specimens treated with certain concentration of fungicide solution

3-10 Test results

The results are shown below in Table 3. Both lower rating value and lower damage value correspond to higher wood fungicide activity. As demonstrated in Table 3, example compounds 1, 4, 5, 6 and 8 show excellent wood fungicide activity.

Table 3

| Average rating value | | | | | | | | | | | | |
|---|---|---|---|---|---|---|---|---|---|---|---|---|
| Fungi | Compound (Example No.) | | | | | | | | | | | |
| | 1 | 2 | 4 | 5 | 6 | 7 | 8 | 9 | 10 | 11 | R | N |
| $A_1$ | 0.0 | 3.0 | 0.0 | 0.0 | 0.0 | 0.2 | 0.3 | 1.7 | 2.7 | 3.0 | 0.0 | 3.0 |
| $A_2$ | 0.0 | 2.0 | 0.0 | 0.0 | 0.0 | 0.2 | 0.0 | 1.0 | 0.3 | 1.8 | 0.0 | 3.0 |
| $A_3$ | 2.0 | 3.0 | 0.0 | 3.0 | 3.0 | 3.0 | 3.0 | 3.0 | 3.0 | 3.0 | 2.0 | 3.0 |
| $A_4$ | 0.0 | 0.0 | 0.0 | 0.0 | 0.0 | 0.0 | 0.0 | 0.3 | 0.0 | 1.7 | 0.0 | 3.0 |
| $A_5$ | 0.0 | 1.3 | 1.0 | 1.3 | 0.0 | 1.8 | 0.2 | 1.5 | 2.2 | 2.8 | 0.0 | 3.0 |
| Sum | 2.0 | 9.3 | 1.0 | 4.3 | 3.0 | 5.2 | 3.5 | 7.5 | 8.2 | 12.3 | 2.0 | 15.0 |
| damage value | 13.3 | 62.2 | 6.7 | 28.9 | 20.2 | 34.4 | 23.3 | 50.0 | 54.4 | 82.2 | 13.3 | 100.0 |

Note. Compounds 1-11 : 0.5% xylene solution
R : Reference compound- $I-C\equiv CCH_2O-CO-NH(CH_2)_3CH_3$, 0.5% xylene solution
N : untreated
$A_1$ : Aspergillus Niger (IFO 6341)
$A_2$ : Penicillium funiculosum (IFO 6345)
$A_3$ : Rhizopus javanicus (IFO 6354)
$A_4$ : Aureobasidium pullulans (IFO 6353)
$A_5$ : Gliocladium virens (IFO 6355)

Test example 4 Anti-termite activity

This test was conducted on the basis of the method described in Specification No.11 of Japan Wood Preserving Association, enacted in 1981 and titled "The test methods of anti-termite activity for anti-termite agent used in the coating, spraying and dipping treatment, (1) Laboratory test methods".

4-1 The termite to be used

The termite to be used was Coptofermes formosanus SHIRAKI ("ieshiroari").

4-2 Contact toxicity

4-2-1 The treated quartz sand was prepared as follows : The quartz sand, which passed through a sieve with 60 mesh screen, of 3 g was mixed with the BACTRO-AGAR solution of 1 ml with good agitation. Then, water content was adjusted to 5 %.

4-2-2 The test quartz sand was prepared as follows : The anti-termite agent solution of intended concentration (ppm) was added to the above treated quartz sand, then was mixed, and then was allowed to stand for 14 days to vaporize solvents.

4-2-3 The test quartz sand of 4 g were put into a Petri dish (6 cm in diameter) sterilized with dry heat.

4-2-4 Ten termite workers were randomly sampled from their nest, and then put on the quartz sand.

4-2-5 Each Petri dish was placed in a vessel containing water in the bottom, and then the vessel was allowed to stand in the constant temperature room at 28 ± 2 °C for 14 days. The number of the dead termites was observed and recorded. The first day : every 2 hours for 8 hours. After the second day : every 24 hour.

4-2-6 Test was repeated three times for each concentration of anti-termite agent solution.

4-3 Test results

4-3-1 Each time when observing, the number of healthy termites and dead termites were recorded.
4-3-2 The results are shown below in Table 4. As demonstrated in Table 4, the compounds of the present invention have slow but strong termite toxicity.

Table 4

| Death rate (%) | | | | | | | |
|---|---|---|---|---|---|---|---|
| | Compound (Example No.) | | | | | | |
| | 1 | | 4 | | 8 | | N |
| concentration ( ppm) | 100 | 1000 | 100 | 1000 | 100 | 1000 | --- |
| term of contact 1 day | 0 | 0 | 0 | 0 | 0 | 0 | 0 |
| 2 days | 0 | 50 | 0 | 30 | 0 | 0 | 0 |
| 3 days | 30 | 100 | 30 | 100 | 20 | 70 | 0 |
| 5 days | 100 | 100 | 100 | 100 | 100 | 100 | 0 |
| Note. N : untreated | | | | | | | |

**Claims**

1. A compound of the formula:

   $I\text{-}C{\equiv}C\text{-}CO\text{-}NR_1R_2$

   wherein $R_1$ is a hydrocarbon group which may be substituted or an aromatic heterocyclic group which may be substituted, $R_2$ is a hydrogen atom, a hydrocarbon group which may be substituted or an aromatic heterocyclic group which may be substituted, or $R_1$ and $R_2$ together with the adjacent nitrogen atom form a ring.

2. The compound according to claim 1, wherein the hydrocarbon group is $C_{1-24}$alkyl, $C_{3-8}$cycloalkyl, $C_{2-24}$alkenyl, $C_{2-24}$alkynyl, $C_{6-18}$aryl or $C_{6-18}$ar-$C_{1-24}$alkyl group.

3. The compound according to claim 1, wherein the aromatic heterocyclic group is 5- to 10-membered aromatic heterocyclic group having 1 to 4 nitrogen atoms and/or oxygen atoms and/or sulfur atoms as ring-constituting atoms.

4. The compound according to claim 1, wherein $R_1$ is $C_{1-18}$alkyl group.

5. The compound according to claim 1, wherein $R_1$ is phenyl which may be substituted.

6. The compound according to claim 1, wherein $R_2$ is a hydrogen atom.

7. The compound according to claim 1, wherein $R_1$ is $C_{4-10}$ alkyl group and $R_2$ is a hydrogen atom.

8. The compound according to claim 1, wherein $NR_1R_2$ is piperidyl, piperadinyl or morpholinyl which may be substituted.

9. The compound according to claim 1, which is N-(n-butyl)-3-iodopropiolamide.

10. The compound according to claim 1, which is N-(tert-butyl)-3-iodopropiolamide.

11. A composition for controlling noxious organisms which comprises the compound according to claim 1 together with a carrier.

**12.** The composition according to claim 11, which is wood preservative.

European Patent Office

# EUROPEAN SEARCH REPORT

Application Number

EP 92 11 7193

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | DATABASE WPIL<br>Section Ch, Week 6800,<br>Derwent Publications Ltd., London, GB;<br>Class A, AN 67-8919H<br>& JP-B-44 031 220 (SANKYO CO. LTD.)<br>* abstract * | 1-16 | C07C233/09<br>A61K31/16<br>A01N37/18<br>C07D295/185<br>A61K31/445<br>A01N43/40<br>C07D333/20 |
| A,D | CHEMICAL ABSTRACTS, vol. 81, no. 21,<br>25 November 1974, Columbus, Ohio, US;<br>abstract no. 135429q, 'DIODOACETYLENIC OR IODOACETYLENIC COMPOUNDS'<br>page 370 ;<br>* abstract *<br>& SU-A-436 042 (FILINOV YU.P. ET AL.) | 1-12 | |
| A | US-A-3 657 340 (F. JOHNSON ET AL.)<br>* the whole document * | 1-16 | |
| A | EP-A-0 057 058 (SANKYO COMPANY LIMITED)<br>* claims * | 1-16 | |
| A | EP-A-0 140 270 (BAYER AG)<br>* claims * | 1-16 | TECHNICAL FIELDS SEARCHED (Int. Cl.5) |
| P,X | PATENT ABSTRACTS OF JAPAN<br>vol. 15, no. 436 (C-882)1991<br>& JP-A-31 84 960 ( ISHIHARA SANGYO KAISHA LTD )<br>* abstract * | 1-16 | C07C<br>C07D |

The present search report has been drawn up for all claims

| Place of search | Date of completion of the search | Examiner |
|---|---|---|
| THE HAGUE | 12 MARCH 1993 | SANCHEZ Y GARCIA J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)